# FASCICULE DE BREVET EUROPEEN

(11) **EP 2 391 299 B1**
(45) Date de publication et mention de la délivrance du brevet: **17.01.2018**
(21) Numéro de dépôt: 09736440.0
(22) Date de dépôt: 04.09.2009
(51) Int. Cl.: A61F 2/00

(54) **PLAQUE IMPLANTABLE POUR LA RÉFECTION DE PAROIS**
IMPLANTIERBARE PLATTE ZUR WIEDERHERSTELLUNG VON WÄNDEN
IMPLANTABLE PLATE FOR RESTORING WALLS

(30) Priorité: 30.01.2009 FR 0950600
(43) Date de publication de la demande: 07.12.2011
(73) Titulaire: Textile Hi-Tec (T.H.T.), 34220 Verreries de Moussans (FR)
(72) Inventeur: HOUARD, William, 81270 Labastide Rouairoux (FR); BERTOLASO, Walter, 82000 Montauban (FR)
(74) Mandataire: Cabinet Beau de Loménie
(86) Numéro de dépôt international: PCT/FR2009/001066
(87) Numéro de publication internationale: WO 2010/086515

(56) Documents cités:
- EP-A- 1 384 450
- WO-A-03/026530
- WO-A-03/099160
- FR-A- 2 914 179
- US-A1- 2007 250 147

## Description

La présente invention concerne une plaque prothétique c'est-à-dire une plaque qui est implantable pour la réfection de parois, notamment dans le traitement des hernies.

Dans le domaine chirurgical, on utilise le terme plaque pour désigner de manière générale une pièce souple, dans un matériau biocompatible et généralement poreux, pièce qui est mise en oeuvre pour la confection d'une prothèse, notamment destinée à renforcer une paroi défectueuse. La prothèse en elle-même peut être réalisée à partir d'une seule plaque comme dans le document FR.2.712.177, ou à partir de deux plaques comme dans les documents US.A.4.769.038 et EP.0.836.838 ou même de trois plaques comme dans le document EP.A.0.719.527. La plaque en elle-même peut être formée d'une seule couche, notamment d'un tissu de simple épaisseur comme dans le document FR.2.712.177 ou être composée de plusieurs couches comme dans le document EP.1.567.205.

Pour une parfaite efficacité de la prothèse, il importe que celle-ci reste bien en place dans la position qui lui a été donnée lors de son implantation. Pour ce faire, le praticien réalise la fixation de la prothèse aux structures environnantes à l'aide d'agrafes ou de fils de suture, comme dans le document US.A.4.769.038. Cette fixation, qui est réalisée sous tension, peut provoquer pour le patient une sensation désagréable, voire des douleurs lors de certains mouvements. De plus la pose d'agrafes ou de fils de suture prolonge la durée d'intervention du chirurgien pour la pose proprement dite de la prothèse. De plus les agrafes ou les fils de suture peuvent être une source d'inflammation.

On connaît par le document FR.2.776.179 une plaque chirurgicale composite, qui comporte une couche interne lisse et une couche externe rugueuse, la rugosité de ladite couche étant censée permettre l'accrochage des tissus organiques pour compenser les effets de glissement relatifs de la plaque et des tissus adjacents. Dans ce document FR.2.776.179, la couche externe est de préférence une couche de textile fibrillaire non tissée, notamment constituée de fils de polyamide ou de polypropylène biocompatible. Il est précisé, dans ce document, qu'une telle couche externe permet une bonne colonisation par les tissus organiques en contact avec la plaque.

Cependant le demandeur est d'avis que la simple structure de la couche externe, à savoir une structure fibrillaire non-tissée, n'est pas de nature à éviter le déplacement de la plaque dans la période qui suit l'implantation et qui précède la colonisation tissulaire.

C'est l'objet de la présente invention que de proposer une plaque implantable pour la réfection de parois qui pallie les inconvénients des plaques précitées d'une part en ce qu'elle ne nécessite pas une fixation par agrafage ou suturage et d'autre part qui assure de manière fiable le maintien en place de la prothèse dans la période qui suit l'implantation de ladite prothèse par le chirurgien.

Le document WO03/099160A1 décrit un implant médical qui comporte un film embossé éventuellement associé à une structure de base qui peut être textile. Certes il est mentionné dans ce document que les bossages formés dans ce film peuvent être conçus pour augmenter ou pour réduire la friction de l'implant dans le corps, ce qui peut être utilisé soit pour la fixation de l'implant soit pour augmenter sa mobilité. Mais aucune indication n'est donnée quant à la structure particulière du film qui permet d'obtenir une telle augmentation ou réduction de la friction.

De manière connue, la plaque implantable de la présente invention comporte un support textile et présente des protubérances. Elle se caractérise en ce que les protubérances sont formées dans le support textile lui-même, sur au moins l'une de ses faces. Ainsi, selon la disposition particulière de la présente invention, lorsque la plaque est implantée, la face du support textile qui comporte les protubérances vient en contact avec les tissus organiques et les protubérances qui sont constituées par les fibres ou filaments du support textile forment autant de zones de friction entre la plaque et lesdits tissus, créant des forces de frottement empêchant le déplacement de ladite plaque par rapport auxdits tissus.

Dans un mode préféré de réalisation, les protubérances ont une configuration conique, une telle configuration facilitant la pénétration des protubérances dans les tissus organiques. Cette configuration n'est cependant pas exclusive, les protubérances pouvant avoir notamment une forme ondulatoire ou cylindrique.

Selon une variante de réalisation, le support textile comporte des perforations, lesquelles sont principalement destinées à favoriser le drainage des fluides corporels et éventuellement à favoriser la colonisation tissulaire dans le cas où la structure elle-même du support textile n'est pas suffisamment ouverte. De préférence ces perforations sont disposées au niveau des protubérances en sorte que les contours de la perforation forment des arêtes qui augmentent l'effet de friction de la protubérance proprement dite.

De préférence lorsque la protubérance possède un axe de symétrie, la perforation est un orifice centré sur cet axe. Ainsi, dans le cas où la protubérance a une configuration conique, la présence de la perforation fait qu'en réalité la protubérance a une forme de tronc de cône. Le support textile est constitué au moins en partie de fibres ou filaments thermoplastiques et chaque protubérance est formée dans une zone déterminée du support textile par déformation de la structure du support et par thermosoudage d'au moins certaines desdites fibres ou filaments de ladite zone. Par exemple la protubérance peut être formée à l'intérieur d'une portion périphérique, notamment annulaire, de ladite zone dans laquelle lesdites fibres ou filaments sont thermo-soudés. Le thermo-soudage des fibres ou filaments constitutifs de la portion périphérique assure à ladite portion une certaine rigidité, ce qui permet de former la protubérance en repoussant vers l'extérieur du plan du support textile et donc en déformant la structure du support textile, formé de fibres ou filaments non thermo-soudés, qui se trouve à l'intérieur de ladite portion périphérique.

De préférence, lorsque le support textile comporte des perforations, celles-ci sont centrées par rapport à la portion périphérique de la zone déterminée.

Cependant, de préférence, la protubérance est formée par déformation et par thermosoudage de toutes les fibres ou filaments de la zone déterminée du support textile. Par exemple la déformation intervient par emboutissage entre deux outils complémentaires, mâle et femelle, ayant la configuration souhaitée pour la protubérance et le thermosoudage intervient par application, à l'aide des mêmes outils, d'un traitement apte à réaliser le thermosoudage des fibres ou filaments déformés lors de l'emboutissage. Dans ce cas il y a, dans un premier temps, déformation de la structure initiale du support textile, notamment le déplacement des fibres ou filaments les uns par rapport aux autres jusqu'à obtenir la configuration souhaitée pour la protubérance, et il y a, dans un second temps, thermosoudage des fibres ou filaments dans leur nouvelle disposition. La fusion au moins superficielle des fibres ou filaments qui est développée lors de l'opération de thermosoudage apporte une certaine rigidité à l'ensemble des fibres ou filaments de la protubérance, ce qui augmente ses propriétés frictionnelles et donc le caractère antimigratoire de la plaque.

Le thermo-soudage des fibres ou filaments thermoplastiques est réalisé notamment par ultrasons.

Selon une variante de réalisation, le support textile est un non-tissé qui est thermo-lié par points.

Selon une autre variante de réalisation, le support textile est un tissu du type 3D ou tridimensionnel, constitué de deux couches reliées par des fils de liaison. Cela permet de faire varier plus largement les caractéristiques du support textile, notamment son épaisseur, le choix des composants qui le constituent ainsi que sa porosité. Il peut être obtenu soit par tissage soit par tricotage.

Par tissage, il est obtenu par superposition d'une nappe de fils de chaîne et de plusieurs nappes de fils de trame ou par superposition de plusieurs nappes de fils de chaîne et de fils de trame. La liaison de ces différentes nappes est assurée soit par certains des fils de chaîne d'au moins l'une des nappes de fils de chaîne de la superposition soit par une nappe supplémentaire de fils de chaîne, dite chaîne de liage.

Par tricotage, il est obtenu sur un métier chaîne double fonture, dans lequel la liaison des deux couches respectivement tricotées sur chaque fonture est assurée par une nappe de chaîne supplémentaire qui travaille alternativement sur l'une et l'autre fontures.

Selon une variante de réalisation, au moins certaines protubérances comportent des jours aptes à permettre la colonisation cellulaire de l'implant au niveau desdites protubérances et également un certain drainage des fluides corporels. Cela permet d'éviter de former des perforations dans le support textile.

Lorsque les protubérances sont formées en repoussant les fibres ou filaments constitutifs du support textile, on obtient, lors de cette opération, un déplacement relatif de certaines des fibres ou de certains des filaments susceptible de créer des jours dans au moins certaines protubérances. La présence ou non d'un ou plusieurs jours dans une protubérance, la taille et la configuration de chaque jour sont des paramètres assez aléatoires puisqu'ils dépendent non seulement de la structure, plus ou moins ouverte, du support textile mais aussi de l'emplacement, sur le support textile, de la zone où agissent localement les outils créant la protubérance. Si la structure du support textile est relativement fermée et que les outils agissent dans une zone où il y a une forte densité de fibres ou filaments, la protubérance pourra ne pas contenir de jour. Au contraire, la protubérance sera ajourée si la structure du support textile est elle-même ajourée et que les outils agissent dans une zone comportant déjà au moins une partie de jour.

De préférence, pour augmenter la probabilité d'avoir des protubérances ajourées, on choisit comme support textile un tissu tridimensionnel obtenu par tricotage, comme mentionné ci-dessus, qui permet d'avoir un caractère ajouré plus prononcé et de maîtriser ce caractère ajouré plus facilement que celui obtenu par tissage.

Selon l'application visée, la plaque de la présente invention peut présenter des protubérances sur une seule face ou sur ses deux faces.

Dans un mode de réalisation particulier, la plaque comporte, sur les deux faces du support textile, des protubérances dont la hauteur est sensiblement égale ou supérieure à l'épaisseur (e) dudit support, de préférence inférieure à trois fois ladite épaisseur, notamment de l'ordre de deux fois ladite épaisseur. Si l'épaisseur dépasse trois fois l'épaisseur du support textile, il y a un risque d'agressivité des fibres ou filaments qui constituent lesdites protubérances.

De préférence ces protubérances sont régulièrement réparties, en quinconce et en alternance d'une face à l'autre, à raison de 0,5 à 2 protubérances/cm². Dans un exemple précis de réalisation de protubérances formées par emboutissage à l'intérieur d'une zone annulaire dans laquelle les fibres ou filaments thermoplastiques du support textile étaient thermo-soudées par ultrasons, la zone annulaire avait un diamètre intérieur de l'ordre de 2 à 5 mm et une largeur de l'ordre de 0,5 mm.

Selon une variante de réalisation, le support textile est imprégné, par exemple de collagène qui favorise la colonisation cellulaire ou encore de polyuréthanne, ladite imprégnation étant apte à conférer à la plaque une certaine mémoire de forme. Le but est de permettre que la plaque puisse être enroulée sur elle-même pour son introduction dans un trocart et se déployer spontanément lors de sa libération du trocart.

Selon une variante de réalisation, le support textile comporte sur l'une de ses faces une enduction anti-adhérente, en particulier sur la face ne comportant pas de protubérances antimigratoires. On entend par enduction anti-adhérente une enduction qui est apte à éviter ou tout au moins à limiter fortement les adhérences entre la plaque et les parties du corps contre laquelle elle vient en contact, une fois implantée, notamment les viscères. Il peut s'agir d'une enduction de collagène, de polysaccharide ou autre biopolymère résorbable ou non.

L'implantation d'une prothèse quelle qu'elle soit doit pouvoir faire l'objet d'un suivi par le chirurgien, de manière à vérifier le comportement de la prothèse dans le temps. Dans le document FR.2.712.177, la prothèse est constituée d'un tissu de simple épaisseur résultant d'un tissage ou tricotage de fils polyester multibrins de manière à former une structure aérée à mailles carrées ou rectangulaires. Pour augmenter la rigidité de cette prothèse, tout en constituant un repaire radiologique, les fils en polyester sont combinés en chaîne et/ou en trame avec des fils métalliques. Ainsi la présence de ces fils métalliques permet de suivre le comportement de la prothèse à l'occasion de contrôles radioscopiques périodiques.

Toutefois la présence de fils métalliques dans la plaque prothétique peut être un inconvénient, notamment du fait qu'elle augmente la rigidité de celle-ci et qu'elle augmente son coût de fabrication. De plus il n'est guère envisageable d'introduire des fils métalliques dans une structure autre que tissée ou tricotée. Pour pallier ces inconvénients, la plaque prothétique de la présente invention comporte un marquage radio-opaque sur toute ou partie de la surface du support textile.

Dans une variante de réalisation, ce marquage radio-opaque résulte de l'imprégnation localisée du support textile par une composition siliconée comportant une charge radio-opaque, notamment une charge de sulfate de baryum ou de tantale.

Selon un mode de réalisation, le marquage se présente sous la forme de lignes pour constituer un quadrillage régulier.

Dans un exemple précis et préféré, le pas du quadrillage est compris entre 2 et 45 mm, de préférence de l'ordre de 15 mm.

La présente invention sera mieux comprise à la lecture de la description d'exemples de réalisation d'une plaque prothétique comportant des protubérances anti-migratoires, illustrés par le dessin annexé dans lequel :
La figure 1 est une représentation schématique en vue de dessus d'une portion de plaque prothétique comportant deux protubérances tronconiques,
La figure 2 est une représentation schématique en coupe de la plaque prothétique de la figure 1 selon le plan II-II,
La figure 3 est une représentation schématique en vue de dessus d'une plaque prothétique présentant des protubérances en relief sur ses deux faces,
La figure 4 est une représentation schématique en vue de dessus de la plaque de la figure 3 comportant un marquage radio-opaque sous forme d'un quadrillage passant entre les protubérances, et
La figure 5 est une photographie prise au microscope illustrant une protubérance formée dans un tricot tridimensionnel.

De manière générale selon l'invention, la plaque prothétique 1, destinée à la réfection de parois notamment dans le domaine des hernies, comporte un support textile 2 dont au moins l'une des faces 2a est surmontée de protubérances anti-migratoires 3 formées à partir des fibres ou filaments composant le support textile lui-même.

Ce sont les fibres ou filaments qui, venant en contact direct avec les tissus organiques, confèrent auxdites protubérances un effet anti-migratoire, empêchant la migration de la plaque 1 une fois que celle-ci a été implantée par le chirurgien sans être fixée d'une quelconque manière, que ce soit par des moyens mécaniques tels que des agrafes ou des fils de suture ou par tout autre moyen.

Le support textile 2 est, dans le premier exemple qui va être décrit, un non-tissé, formé par l'enchevêtrement de fibres ou de filaments thermoplastiques qui sont solidarisés les uns aux autres par liage, plus précisément par thermoliage par points, obtenu par passage d'une nappe de fibres ou de filaments entre deux cylindres chauffants gravés. Il s'agit en particulier d'un non-tissé faisant de 45 à 100g/m², fait à partir de filaments de polypropylène. Dans cet exemple le liage a une densité de 36 points/cm², chaque point faisant de l'ordre de 0,1 mm².

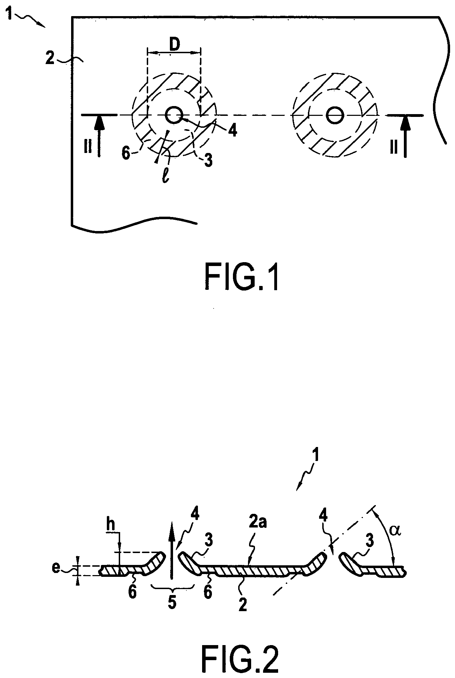

Dans le mode de réalisation illustré aux figures 1 et 2, chaque protubérance 3 a la forme d'un tronc de cône, la petite base ouverte du tronc de cône correspondant à une perforation 4, c'est-à-dire un trou débouchant, formé dans le support textile 2. L'angle α d'inclinaison du tronc de cône, par rapport au plan général du support textile 2, est de l'ordre de 45° dans l'exemple illustré à la figure 2. Cette valeur n'est pas limitative. Dans le cas où cet angle α est de 90°, la protubérance a alors une configuration qui n'est plus tronconique mais cylindrique. D'autres configurations sont bien sûr possibles pour autant que cette configuration et le nombre de protubérances permettent d'atteindre le résultat recherché à savoir de créer sur la surface 2a du support textile 2 autant de points de friction augmentant le coefficient de frottement entre la plaque 1 et les tissus organiques contre lesquels la face 2a de la plaque 1 vient s'appliquer lorsque celle-ci est implantée.

Les protubérances 3 pourraient éventuellement être formées lors de la fabrication du support textile 2. Cependant par simplification elles sont formées sur le support textile déjà réalisé en repoussant vers l'extérieur selon la flèche F de la figure 2 les fibres ou filaments constitutifs dudit support 2 dans une zone localisée 5.

Dans un mode précis de réalisation, cette zone localisée 5 a été circonscrite par une portion périphérique, notamment annulaire 6 dans laquelle les fibres ou filaments constitutifs du support textile 2 ont été thermo-soudées notamment par ultrasons. Ainsi les fibres ou filaments qui sont thermo-soudées dans ladite portion 6 n'ont pas tendance à se déplacer lorsqu'on repousse selon la flèche F les fibres ou filaments non thermo-soudées qui se trouvent à l'intérieur de cette portion périphérique 6. Le thermo-soudage apporte une certaine consolidation du support textile autour de chaque protubérance 3. Cet effet de consolidation, permettant que seules les fibres ou filaments de la zone localisée 5 puissent se déplacer pour former la protubérance 3 est obtenu quelle que soit la forme de la zone périphérique, que celle-ci ait la forme d'un anneau comme dans l'exemple illustré ou toute autre forme.

La configuration de la protubérance 3 est, dans ce cas, fonction de l'outil servant au repoussage ou emboutissage du support textile 2 dans la zone localisée 5. Comme indiqué ci-dessus cette configuration peut être tronconique comme dans l'exemple illustré, cylindrique, voire conique s'il n'y a pas de perforation 4 ou encore ondulatoire, formant un effet de vague ayant non pas un axe de symétrie comme pour les configurations tronconique, conique ou cylindrique mais un plan de symétrie.

Les perforations 4, pratiquées dans le support textile 2, ont pour fonction première de faciliter d'une part le drainage des fluides corporels venant au contact de la plaque et d'autre part la colonisation tissulaire de la plaque, en particulier lorsque celle-ci a une structure microporeuse, comme c'est le cas d'un non-tissé thermo-lié. C'est cette colonisation qui permet d'obtenir une fixation définitive de la plaque 1 dans un délai qui est généralement de quinze jours après l'implantation. La fonction seconde de ces perforations est d'augmenter, par les arêtes qu'elles forment lorsqu'elles sont disposées au niveau des protubérances 3, autant de points complémentaires de friction, contribuant à l'effet anti-migratoire des protubérances elles-mêmes.

La plaque prothétique 1 de la figure 1 est par exemple obtenue en deux étapes successives. La première étape consiste, partant d'un support textile de grande dimension, en deux opérations simultanées de découpe et de perforation par emboutissage. L'opération de découpe permet de donner à la plaque 1 ses dimensions extérieures, par exemple un rectangle de 17 cm x 15 cm. L'opération de perforation permet de réaliser autant de trous débouchants que de perforations 4 souhaitées en nombre et en dimension, par exemple des perforations circulaires faisant de l'ordre de 1 à 2 mm de diamètre, à raison de 0,5 à 2 perforations/cm². La seconde étape consiste en deux opérations simultanées de thermo-soudage par ultrasons selon la portion annulaire 6 et de repoussage du support textile à l'intérieur de cette portion annulaire 6. Ces deux opérations nécessitent la mise en oeuvre d'un outil ultrasonique comportant une partie mâle et une partie femelle. La plaque prothétique est placée sur la partie femelle. La partie mâle comporte autant de sonotrodes unitaires que de perforations, chaque sonotrode unitaire venant en appui sur la plaque prothétique dans la portion annulaire et comportant un prolongement central formant embout de repoussage. Le placement de la plaque prothétique 2 sur la partie femelle est tel que chaque perforation 4 est centrée par rapport à une sonotrode annulaire unitaire et à son embout de repoussage. Lors de l'application de la partie mâle sur la partie femelle, les sonotrodes unitaires réalisent la fusion localisée des fibres ou filaments de la prothèse prothétique dans la portion annulaire 6 et l'embout de repoussage déforme la structure du support textile se trouvant à l'intérieur de cette portion annulaire 6, déplaçant les fibres ou filaments non thermosoudés pour former les protubérances 3.

La hauteur h de chaque protubérance est, dans l'exemple illustré à la figure 2, sensiblement de l'ordre de l'épaisseur e de la plaque prothétique 2. En pratique cette hauteur h est de préférence de l'ordre de deux fois cette épaisseur e, n'étant normalement pas supérieure à trois fois cette épaisseur e pour éviter les risques d'agressivité à l'égard des tissus organiques.

Dans le mode de réalisation illustré aux figures 1 et 2, seule la face 2a de la plaque prothétique 2 est surmontée de protubérances 3.

Cependant il peut être souhaité, notamment lorsqu'il s'agit de la réfection des hernies par voie chirurgicale, que la plaque 1 soit pourvue de protubérances anti-migratoires sur ses deux faces.

Si l'on veut mettre en oeuvre la même technique ultrasonique que ci-dessus, le même outil peut être utilisé pour former les zones annulaires et les protubérances sur les deux faces en deux étapes successives, la première pour former les protubérances et les zones annulaires correspondant auxdites protubérances sur une face et la seconde pour former les protubérances et les zones annulaires correspondantes sur l'autre face, après retournement du support textile.

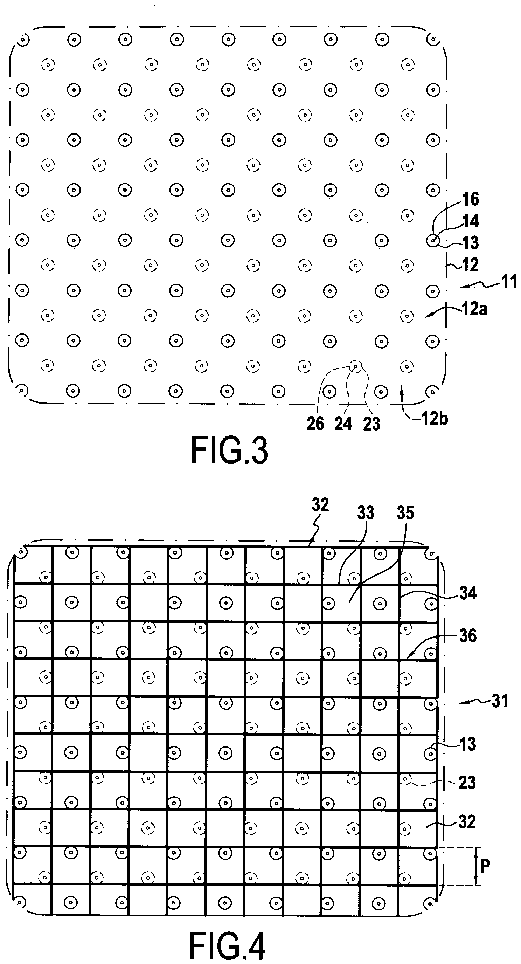

Sur la figure 3 est représenté un exemple de réalisation d'une plaque prothétique 11 dont le support textile 12 est pourvu sur sa face supérieure 12a de protubérances 13. Chaque protubérance 13 est délimitée par une portion périphérique 16 et comporte une perforation centrale 14. Cette même plaque prothétique 11 comporte, sur son autre face interne 12 b, des protubérances 23, délimitées par une portion périphérique 26, en pointillés sur la figure 3 et comportant également une perforation centrale 24. Toutes ces protubérances 13 et 23 sont régulièrement réparties en quinconce et en alternance d'une face 12a à l'autre 12b. En pratique sur les deux faces 12a et 12b, les protubérances 13, 23 forment des alignements parallèles à la fois longitudinaux et transversaux, ces alignements étant décalés d'une face à l'autre d'une distance qui est égale à la moitié de l'écartement entre deux protubérances adjacentes.

Sur les deux faces 12a et 12b, les protubérances 13, 23 ont sensiblement la même hauteur h.

La portion périphérique annulaire 6, 16, 26 a de préférence un diamètre intérieur D qui est de l'ordre de 2 à 5 mm et une largeur l qui est de l'ordre de 0,5 mm.

Sur la figure 4 on a représenté une plaque 31 qui comporte sur ses deux faces des protubérances 13, 23 identiques à celles de l'exemple de la figure 3. Cette plaque prothétique 31 comporte de plus un marquage radio-opaque 36 qui est destiné à permettre le suivi radiologique de la plaque prothétique 31 après son implantation. Ce suivi doit permettre la vérification du bon positionnement de la plaque durant la période qui précède la fixation par colonisation tissulaire. De plus, grâce au marquage radio-opaque, le contrôle de la plaque dans le temps est rendu possible par réalisation de radiographies comparatives et mesure des intervalles de la trame radio-opaque, permettant en particulier d'évaluer un éventuel déplacement de la plaque durant son vieillissement. Le suivi radiologique doit également permettre le repérage de l'emplacement de la plaque en vue d'une nouvelle intervention sur une autre pathologie nécessitant le passage chirurgical par la zone où se trouve ladite plaque. Enfin il doit permettre l'étude, par un examen simple et peu coûteux, de l'évolution dans le temps du support textile en terme de rétraction et de vieillissement, notamment l'étude comparative de la trame radio-opaque permet, en cas de récidive, de mieux en appréhender la cause.

Le marquage radio-opaque se présente sous la forme de lignes 33, 34 pour constituer un quadrillage régulier. Le pas P du quadrillage est compris entre 2 et 45 mm, de préférence de l'ordre de 15 mm pour une plaque rectangulaire faisant 17 cm x 15 cm. Dans l'exemple illustré à la figure 4, les lignes 33,34 passent entre les protubérances 13, 23 ; ceci n'est cependant pas limitatif.

Grâce à la disposition en quadrillage régulier de ce marquage, il est possible, lors de l'examen radioscopique, de vérifier s'il y a ou non une évolution dans les distances séparant les lignes adjacentes du quadrillage et par conséquent de constater une éventuelle rétraction du support textile dans lequel est formée la plaque.

Le marquage radio-opaque proprement dit peut en particulier être obtenu par imprégnation localisée, selon les lignes du quadrillage ou selon tout autre motif du support textile (32), par une composition siliconée comportant une charge radio-opaque, charge qui peut notamment être du sulfate de baryum ou du tantale.

Sur la figure 4 le quadrillage 32 est formé de lignes 33 longitudinales et de lignes 34 transversales, délimitant des carrés 35, chaque carré contenant une ou deux protubérances 13, 23. De plus le quadrillage 32 occupe toute la surface de la plaque 31. Ces dispositions particulières ne sont pas exclusives, notamment le quadrillage pouvant n'occuper qu'une partie de la plaque 31.

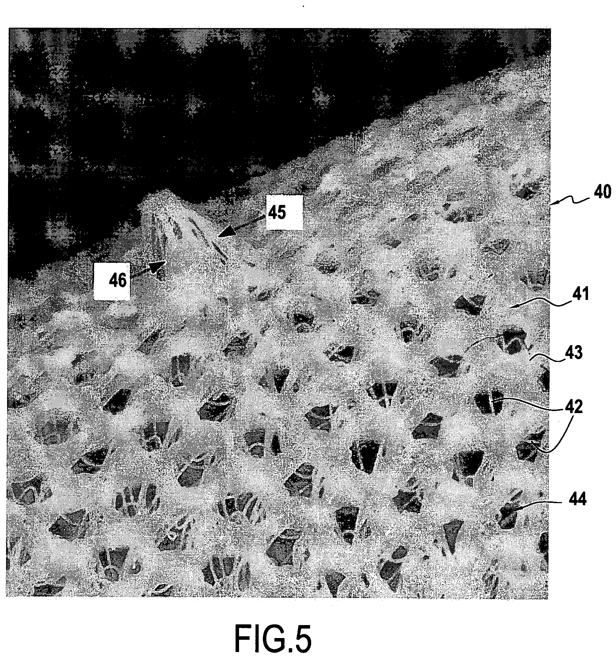

Dans un second exemple de réalisation, qui va être décrit en référence à la figure 5, le support textile 41 de la plaque 40 est un tissu du type 3D ou tridimensionnel, constitué de deux couches reliées par des fils de liaison 42 obtenu par tricotage sur un métier chaîne double fonture. A titre d'exemple non limitatif, il a été réalisé à partir de multi-filaments en polyester pour ce qui est des deux couches 43 et de mono-filaments en polyester pour ce qui est des fils de liaison 42. Il présente une structure macroporeuse en nid d'abeille, avec des mailles hexagonales, formant des jours ou pores réguliers 44.

La technique de formation des protubérances est la même que celle qui a été décrite ci-dessus, exception faite qu'il n'y a pas de perforations formées dans le support textile 41 préalablement à la formation des protubérances et que le thermosoudage intervient sur toutes les fibres ou filaments se trouvant dans la zone déterminée où va être formée la protubérance. Ceci est obtenu par emboutissage et thermosoudage à l'aide de deux outils complémentaires, mâle et femelle, ayant la configuration souhaitée pour la protubérance, avec ou sans portion périphérique. Le thermosoudage intervient par application d'un traitement ultrasonique, à l'aide de ces deux outils qui sont en l'occurrence des sonotrodes, sur les fibres ou filaments qui sont déformés et comprimés entre les deux outils mâle et femelle. Dans ce cas il y a, dans un premier temps, déformation de la structure initiale du support textile, notamment le déplacement des fibres ou filaments les uns par rapport aux autres jusqu'à obtenir la configuration souhaitée pour la protubérance, et il y a, dans un second temps, thermosoudage des fibres ou filaments dans leur nouvelle disposition. La fusion au moins superficielle des fibres ou filaments qui est développée lors de l'opération de thermosoudage apporte une rigidité certaine à l'ensemble des fibres ou filaments de la protubérance, ce qui augmente ses propriétés frictionnelles et donc le caractère antimigratoire de la plaque.

On voit clairement sur la figure 5 que la protubérance 45 comporte, sur sa paroi sensiblement tronconique, des jours 46 qui résultent au moins en partie de la déformation, notamment l'allongement, des jours 44 existant dans la structure du tricot 3D 41, déformation qui intervient lors de l'emboutissage dudit tricot 41 par l'outil mâle de repoussage. Ces jours 46 permettent la colonisation tissulaire de la plaque 41 au niveau des protubérances 45 elles-mêmes, qui sont les plus immédiatement en contact avec les tissus organiques. De ce fait, l'ancrage de la plaque 40 dû à la colonisation tissulaire s'avère plus rapide que si cette colonisation n'intervenait pas au niveau des protubérances 45 mais uniquement des autres jours 44 du tricot tridimensionnel 41. Toutes les protubérances ne comportent pas obligatoirement des jours et les jours présents dans les protubérances n'ont pas tous la même taille et la même configuration puisque cela dépend de la structure du support textile dans la zone déterminée dans laquelle intervient de manière aléatoire l'action des outils de repoussage pour former la protubérance.

La dimension la plus grande des jours dans chaque protubérance peut être de l'ordre de 1 à 1,5 mm.

Dans la description ci-dessus il n'a été question que du support textile 2, comme élément formant la plaque prothétique. Ceci n'est pas exclusif. Le support textile peut comporter une imprégnation dont le but est de conférer à la plaque une certaine mémoire de forme, permettant que celle-ci puisse être enroulée sur elle-même pour son introduction dans un trocart et se déployer spontanément lors de sa libération du trocart. Il peut s'agir d'une imprégnation de collagène, qui présente l'avantage d'accélérer la colonisation tissulaire. Il peut aussi s'agir d'une imprégnation de polyuréthanne.

Le support textile peut également comporter sur l'une de ses faces une enduction anti-adhérente, en particulier sur la face ne comportant pas de protubérances antimigratoires. On entend par enduction anti-adhérente une enduction qui est apte à éviter ou tout au moins à limiter fortement les adhérences entre la plaque et les parties du corps contre laquelle elle vient en contact, une fois implantée, notamment les viscères. Il peut s'agir d'une enduction de collagène, de polysaccharide ou autre biopolymère résorbable ou non.

Dans le cas où le support textile est imprégné ou comporte une enduction anti-adhérente, la formation des protubérances telle que décrite ci-dessus peut intervenir soit sur le support textile seul avant imprégnation ou avant enduction soit respectivement sur le support textile déjà imprégné ou sur le support textile déjà revêtu de son enduction.

## Revendications

1. Plaque (1) implantable pour la réfection de paroi, comportant un support textile (2,41) et présentant des protubérances ayant une configuration conique ou cylindrique, **caractérisée en ce que** les protubérances (3) sont formées dans au moins l'une des faces (2a) dudit support textile (2), les fibres ou filaments qui constituent lesdites protubérances leur conférant un effet anti-migratoire, **en ce que** le support textile est constitué au moins en partie de fibres ou filaments thermoplastiques et chaque protubérance (3,45) est formée dans une zone déterminée (5) du support textile (2,41) par déformation de la structure du support (2,41) et par thermosoudage d'au moins certaines desdites fibres ou filaments de ladite zone (5), et **en ce que** ledit support textile est un non-tissé qui est thermolié par points ou un tissu de type tridimensionnel constitué de deux couches reliées par des fils de liaison, notamment obtenu soit part tissage soit par tricotage.

2. Plaque selon la revendication 1, **caractérisée en ce que** le support textile (2) comporte des perforations (4), notamment disposées au niveau des protubérances (3).

3. Plaque selon l'une ou l'autre des revendications 1 et 2, **caractérisée en ce que** chaque protubérance (3,45) est formée par emboutissage à l'intérieur de ladite zone déterminée (5) dans laquelle tout ou partie desdites fibres ou filaments sont thermo-soudés, notamment par ultrasons.

4. Plaque selon les revendications 2 et 3, **caractérisée en ce que** la perforation (4) est centrée par rapport à une portion périphérique (6) de la zone déterminée (5), dans laquelle portion les fibres ou filaments sont thermosoudés.

5. Plaque selon la revendication 4, **caractérisée** en que la portion périphérique (6) est une portion annulaire ayant un diamètre intérieur de l'ordre de 2 à 5 mm et, de préférence, une largeur (I) de l'ordre de 0,5 mm

6. Plaque (11) selon l'une des revendications 1 à 5, **caractérisée en ce qu'**elle comporte, sur les deux faces (12a,12b) du support textile (12), des protubérances (13) dont la hauteur (h) est sensiblement égale ou supérieure à l'épaisseur (e) dudit support, de préférence inférieure à trois fois ladite épaisseur, notamment de l'ordre de deux fois ladite épaisseur.

7. Plaque selon l'une ou l'autre des revendications 1 à 6, **caractérisée en ce qu'**elle comporte des protubérances (13,23), régulièrement réparties en quinconce et en alternance d'une face à l'autre, à raison de 0,5 à 2 protubérances par cm².

8. Plaque (40) selon l'une des revendications 1,3, 5 à 7 **caractérisée en ce que** le support textile est un tricot ajouré obtenu par tricotage sur métier chaîne double fonture.

9. Plaque selon l'une des revendications 1, 3, 5 à 8, **caractérisé en ce qu'**au moins certaines des protubérances (45) présentent des jours (46), aptes à permettre la colonisation tissulaire desdites protubérances.

10. Plaque selon l'une des revendications 1 à 9 **caractérisée en ce que** le support textile est imprégné, notamment de collagène ou de polyuréthanne.

11. Plaque selon l'une des revendications 1 à 10 **caractérisée en ce que** le support textile comporte sur l'une de ses faces une enduction anti-adhérente, en particulier sur la face ne comportant pas de protubérance antimigratoire.

12. Plaque (31) selon l'une ou l'autre des revendications 1 à 11, **caractérisée en ce qu'**elle comporte un marquage radio-opaque (35) sur tout ou partie de la surface du support textile (32), résultant notamment de l'imprégnation localisée du support textile (32) par une composition siliconée comportant une charge radio-opaque, par exemple une charge de sulfate de baryum ou de tantale.

13. Plaque selon la revendication 12, **caractérisée en ce que** le marquage se présente sous la forme de lignes (33,34)-pour constituer un quadrillage régulier, le pas (P) du quadrillage étant notamment compris entre 2 et 45 mm, de préférence de l'ordre de 15 mm.

## Patentansprüche

1. Implantierbare Platte (1) für die Wiederherstellung einer Wand, die einen Textilträger (2, 41) umfasst und Ausstülpungen mit einer konischen oder zylindrischen Ausgestaltung aufweist, **dadurch gekennzeichnet, dass** die Ausstülpungen (3) in wenigstens einer der Seiten (2a) des Textilträgers (2) ausgebildet sind, wobei die Fasern oder Filamente, welche die Ausstülpungen bilden, ihnen einen wanderungshemmenden Effekt verleihen, dass der Textilträger wenigstens teilweise aus thermoplastischen Fasern oder Filamenten besteht und jede Ausstülpung (3, 45) in einem bestimmten Bereich (5) des Textilträgers (2, 41) durch Verformung der Struktur des Trägers (2, 41) sowie durch Warmverschweißen von wenigstens einigen der Fasern oder Filamente des Bereichs (5) gebildet ist, und dass der Textilträger ein Vlies, welches punktweise warmverbunden ist, oder ein Gewebe vom dreidimensionalen Typ ist, bestehend aus zwei Lagen (43), welche durch Verbindungsfäden (42) verbunden sind, insbesondere erhalten entweder durch Weben oder durch Wirken.

2. Platte nach Anspruch 1, **dadurch gekennzeichnet, dass** der Textilträger (2) Durchbrechungen (4), die insbesondere im Bereich der Ausstülpungen (3) angeordnet sind, umfasst.

3. Platte nach dem einen oder anderen der Ansprüche 1 und 2, **dadurch gekennzeichnet, dass** jede Ausstülpung (3, 45) durch Tiefziehen innerhalb des bestimmten Bereichs (5) gebildet ist, in dem alle Fasern oder Filamente oder ein Teil davon insbesondere durch Ultraschall warmverschweißt sind.

4. Platte nach den Ansprüchen 2 und 3, **dadurch gekennzeichnet, dass** die Durchbrechung (4) in Bezug auf einen Umfangsabschnitt (6) des bestimmten Bereichs (5) zentriert ist, in welchem Abschnitt die Fasern oder Filamente warmverschweißt sind.

5. Platte nach Anspruch 4, **dadurch gekennzeichnet, dass** der Umfangsabschnitt (6) ein ringförmiger Abschnitt mit einem Innendurchmesser in der Größenordnung von 2 bis 5 mm und vorzugsweise einer Breite (I) in der Größenordnung von 0,5 mm ist.

6. Platte (11) nach einem der Ansprüche 1 bis 5, **dadurch gekennzeichnet, dass** sie auf beiden Seiten (12a, 12b) des Textilträgers (12) Ausstülpungen (13) umfasst, deren Höhe (h) im Wesentlichen gleich der oder größer als die Dicke (e) des Trägers, vorzugsweise kleiner als die dreifache Dicke, insbesondere in der Größenordnung von der zweifachen Dicke ist.

7. Platte nach dem einen oder anderen der Ansprüche 1 bis 6, **dadurch gekennzeichnet, dass** sie Ausstülpungen (13, 23) umfasst, die von einer Seite zur anderen mit 0,5 bis 2 Ausstülpungen pro cm² versetzt und abwechselnd gleichmäßig verteilt sind.

8. Platte (40) nach einem der Ansprüche 1, 3, 5 bis 7, **dadurch gekennzeichnet, dass** der Textilträger ein durchbrochenes Gewirk ist, das durch Wirken auf einer zweifonturigen Kettenwirkmaschine erhalten wird.

9. Platte nach einem der Ansprüche 1, 3, 5 bis 8, **dadurch gekennzeichnet, dass** wenigstens einige der Ausstülpungen (45) Öffnungen (46) aufweisen, die geeignet sind, die Gewebebesiedlung der Ausstülpungen zu ermöglichen.

10. Platte nach einem der Ansprüche 1 bis 9, **dadurch gekennzeichnet, dass** der Textilträger imprägniert ist, insbesondere mit Kollagen oder mit Polyurethan.

11. Platte nach einem der Ansprüche 1 bis 10, **dadurch gekennzeichnet, dass** der Textilträger auf einer seiner Seiten eine Antihaftbeschichtung aufweist, insbesondere auf der Seite, die keine wanderungshemmende Ausstülpung umfasst.

12. Platte (31) nach dem einen oder anderen der Ansprüche 1 bis 11, **dadurch gekennzeichnet, dass** sie eine röntgenstrahlenundurchlässige Markierung (35) auf der gesamten Oberfläche des Textilträgers (32) oder einem Teil davon umfasst, die insbesondere aus der stellenweisen Imprägnierung des Textilträgers (32) mit einer Silikonzusammensetzung, welche einen röntgenstrahlenundurchlässigen Füllstoff, beispielsweise einen Baryumsulfat- oder Tantal-Füllstoff umfasst, resultiert.

13. Platte nach Anspruch 12, **dadurch gekennzeichnet, dass** die Markierung in Form von Zeilen (33, 34) vorliegt, um ein gleichmäßiges Gitter zu bilden, wobei die Schrittweite (P) des Gitters insbesondere zwischen 2 und 45 mm beträgt, vorzugsweise in der Größenordnung von 15 mm liegt.

## Claims

1. An implantable plate (1) for reconstruction of walls, comprising a textile support (2,41) and having protuberances, said protuberances having a conical or cylindrical configuration, **characterised in that** the protuberances (3) are formed in at least one of the faces (2a) of said textile support (2), the fibres or filaments which constitute said protuberances lending them an anti-migratory effect, **in that** the textile support is constituted at least in part by thermoplastic fibres or filaments and each protuberance (3,45) is formed in a determined zone (5) of the textile support (2,41) by deformation of the structure of the support (2,41) and by thermowelding of at least some of said fibres or filaments of said zone, and **in that** said textile support is a non-woven material which is thermo-bonded at points or, a tissue of three-dimensional type, constituted of two layers (43) joined by connecting threads (42), especially made by weaving or by knitting.

2. The plate according to claim 1, **characterised in that** the textile support (2) comprises perforations (4), especially arranged at the level of the protuberances (3).

3. The plate according to claim 1 or 2, **characterised in that** each protuberance (3, 45) is formed by swaging inside said determined zone (5) in which all or part of said fibres or filaments are thermowelded, especially by ultrasound.

4. The plate according to claims 2 and 3, **characterised in that** the perforation (4) is centred relative to a peripheral portion (6) of the determined zone (5), in which portion the fibres or filaments are thermowelded.

5. The plate according to claim 4, **characterised in that** the peripheral portion (6) is an annular portion having an inner diameter of the order of 2 to 5 mm and, preferably, a width (I) of the order of 0.5 mm

6. The plate (11) according to anyone of the claims 1 to 5, **characterised in that** it comprises, on the two faces (12a, 12b) of the textile support (12), protuberances (13) whereof the height (h) is substantially equal to or greater than the thickness (e) of said support, preferably less than three times said thickness, especially of the order of twice said thickness.

7. The plate according to anyone of the claims 1 to 6, **characterised in that** it comprises protuberances (13, 23), uniformly distributed in a stagger and alternating from one face to the other, at the rate of 0.5 to 2 protuberances per cm².

8. The plate (40) according to anyone of the claims 1, 3, 5 to 7, **characterised in that** the textile support is an open knit obtained by knitting on a double-bed warp knitting machine.

9. The plate according to anyone of the claims 1, 3 and 5, **characterised in that** at least some of the protuberances (45) have openings (46), capable of allowing tissular colonisation of said protuberances.

10. The plate according to anyone of the claims 1 to 9, **characterised in that** the textile support is impregnated, especially with collagen or polyurethane.

11. The plate according to anyone of the claims 1 to 10, **characterised in that** the textile support comprises on one of its faces an anti-adherent coating, in particular on the face not having an anti-migratory protuberance.

12. The plate (31) according to anyone of the claims 1 to 11, **characterised in that** it comprises radio-opaque marking (35) on all or part of the surface of the textile support (32), resulting especially from localised impregnation of the textile support (32) by a silicon composition comprising a radio-opaque charge, for example a charge of barium sulphate or tantalum.

13. The plate according to claim 12, **characterised in that** the marking is in the form of lines (33, 34) to constitute a regular grid, the pitch (P) of the grid being especially comprised between 2 and 45 mm, preferably of the order of 15 mm.
